# EUROPEAN PATENT SPECIFICATION

(11) **EP 0 295 285 B1**
(45) Date of publication and mention of the grant of the patent: **06.04.1994**
(21) Application number: 88900596.3
(22) Date of filing: 28.12.1987
(51) Int. Cl.: C12N 15/18

(54) **METHOD FOR THE PRODUCTION OF BOVINE GROWTH HORMONE USING A SYNTHETIC GENE**
VERFAHREN ZUR HERSTELLUNG EINES RINDERWACHSTUMSHORMONS MITTELS EINES KUNSTGENS
PROCEDE DE PRODUCTION DE L'HORMONE DE CROISSANCE BOVINE PAR L'UTILISATION D'UN GENE SYNTHETIQUE

(30) Priority: 31.12.1986 KR 1171186; 31.12.1986 KR 1171286
(43) Date of publication of application: 21.12.1988
(73) Proprietor: LUCKY, LTD., Seoul 150 (KR)
(72) Inventor: CHO,Joong Myung, Seoul 133 (KR); LEE, Tae, Ho, Daejeon-shi Chungcheongnam 300-31 (KR); CHUNG,Hyun Ho, Kwanak-gu Seoul 151 (KR); LEE, Yong, Beom, Daejeon-shi Chungcheongnam-do 300-01 (KR); LEE, Tae, Gyu, Seoul 132 (KR); PARK,Young Woo, Daejeon-shi Chungcheongnam-do 300-31 (KR); HAN, Kyu, Beom, Daejeon-shi Chungcheongnam-do 300-31 (KR)
(74) Representative: Ben-Nathan, Laurence Albert
(86) International application number: KR8700013
(87) International publication number: WO8805078

(56) References cited:
- EP-A- 0 067 026
- EP-A- 0 068 646
- EP-A- 0 103 395
- EP-A- 0 111 814
- EP-A- 0 112 012
- EP-A- 0 219 814

## Description

The present invention relates to a method for the production of bovine growth hormone by synthetic gene in yeast or E.coli. Bovine growth hormone is used to promote the growth of cattle, to increase the secretion of milk in cows and to elevate tht efficiency of feed.

Synthetic steroids, for example, Estradiol-compudose by Eli Lilly U.S.A. ; Estradiol benzoate-Synovax by Syntax, U.S.A. have been used in order to promote the growth of cattle and elevate the efficiency of feed in the livestock business up to now.

But it has been found in developed nations including the U.S.A. that synthetic steroids remain in the body for a long time after they have been ingested with feed and then have a detrimental influence when such products are consumed by humans, and prohibition of their usage has increased.

On the other hand, because bovine growth hormone does not remain inside the body after being ingested and it has a species-specificity, as it is a naturally occuring bovine protein, it is a desirable material for human beings. However, until early 1980's it has not been readily available to the livestock industry since it is extracted only from bovine pituitary glands and its amount has been largely limited for general use.

The present inventors have discovered that bovine growth hormone may be produced economically and in large quantity in yeast or E.coli by using gene manipulation and accomplished the present invention

### SUMMARY OF THE INVENTION

One object of the present invention is to provide a method for the production of bovine growth hormone by means of using yeast as a host for expression vector.

The other object of the present invention is to provide a method for the production of bovine growth hormone by means of using E.coli as a host for expression vector.

Firstly, the method for production of bovine growth hormone by means of yeast as a host for expression vector comprises ; (a) synthesizing oligonucleotides having SacI, PstI and SalI restriction sites based on amino acid sequence of bovine growth hormone, (b) cloning one fragment with the PstI/SalI sites and the other fragment with the SacI/PstI sites according to the ligation strategy to a vector for E.coli (pUC18) comprising PstI, SacI and SalI restriction sites, respectively, (c) recloning two cloned fragments cut with PstI/SalI and PstI/SacI restriction enzymes to the vector, pUC18, to get bovine growth hormone gene lacking a portion of the N-terminus, (d) inserting the partial bovine growth hormone gene and the N-terminal synthetic adaptor to a vector for E.coli consisting of a promoter and a terminator to make a cassette of promoter-bovine growth hormone gene comprising an initiation amino acid codon-terminator, (e) inserting the cassette to a yeast expression vector and (f) expressing the resultant vector in yeast cells.

The amino acid codon of the N-terminal synthetic adaptor has the following base sequence of 58 mer and 50 mer with NcoI and SacI restriction sites : 
5' CATGGCTTTCCCGGCTATGTCTCTATCTGGTCTATTCGCTAACGCTGTTCT

CGAAAGGGCCGATACAGAGATAGACCA G A T AAGCGATTGCGACAA G A

NcoI site

TCGAGCT 3'

AGC

SacI site
Secondly, the method for the production of bovine growth hormone by using E.coli as a host for expression vector comprises ;
(a) synthesizing oligonucleotides with SacI, PstI and SalI restriction sites based on amino acid sequence of bovine growth hormone, (b) cloning the N-terminal fragment with the SacI/PstI sites and the C-terminal fragment with the PstI/SalI sites according to the ligation strategy to a vector for E.coli(pUC18), respectively, (c) recloning the two cloned fragments cut with PstI/SacI and PstI/SalI restriction enzymes to the vector, pUC18, to get bovine growth hormone gene lacking a portion of the N-terminus, (d) inserting the partial bovine growth hormone gene and the synthetic adaptor to a vector for E.coli to obtain the bovine growth hormone gene ligated with the synthetic adaptor, (e) inserting the obtained bovine growth hormone gene to E.coli expression vector being able to express salmon growth hormone under the control of a trp promoter and (f) expressing the bovine growth hormone in E.coli.

The synthetic adaptor designed to consists of a terminating codon, TAA and an independent SD sequence before the initiation codon, ATG and prevents the formation of the secondary structure of m-RNA between the SD sequence and the initiation codon ATG. The adaptor sequence comprises and upper strand of 73 mer 
(5'-CATG
GAG GAA TTA TAA ATG GCT TTT CCG GCT ATG TCT CTA TCT GGT CTA TTC
GCT AAC GCT GTT CTT CGA GCT-3')
and a lower strand of 65 mer

(3'-CTC CTT AAT ATT TAC CGA AAA GGC CGA TAC AGA GAT AGA CCA GAT
AAG CGA TTG CGA CAA GAA GC-5').

The SacI/SalI fragment of the bovine growth hormone gene of 526 bp and the synthetic adaptor were cloned to vector pSOD [Hallewell, R.A. et al, Nucleic Acid Res. 13, 2017 (1985)] treated with NcoI and SalI restriction enzymes to produce the bovine growth hormone gene ligated with the synthetic adaptor (pSOD BGH). The vector (pSOD BGH) was treated with NcoI and SalI restriction enzymes to separate the bovine growth hormone gene and the separated fragment was inserted to an E.coli expression vector in which a part of the salmon growth hormone gene is removed by XbaI and SalI restriction enzymes, ptrp322HSGH [it is prepared by cloning salmon growth hormone to ptrp322 (Pharmacia Picataway, N.J. 08854, U.S.A; Russel, D.R. & Bennet, G.N. Gene 20, 231(1981)) and applied to Korea by the present applicant on the same date], to produce ptrphs BGH 1-13. The SD sequence and the initiation codon, ATG, the first part of the salmon growth hormone gene were used for expression in E.coli, in which the salmon growth hormone gene of 37 bp from the initiation codon and synthetic adaptor were ligated to bovine growth hormone gene.

### BRIEF DESCRIPTION OF THE DRAWINGS

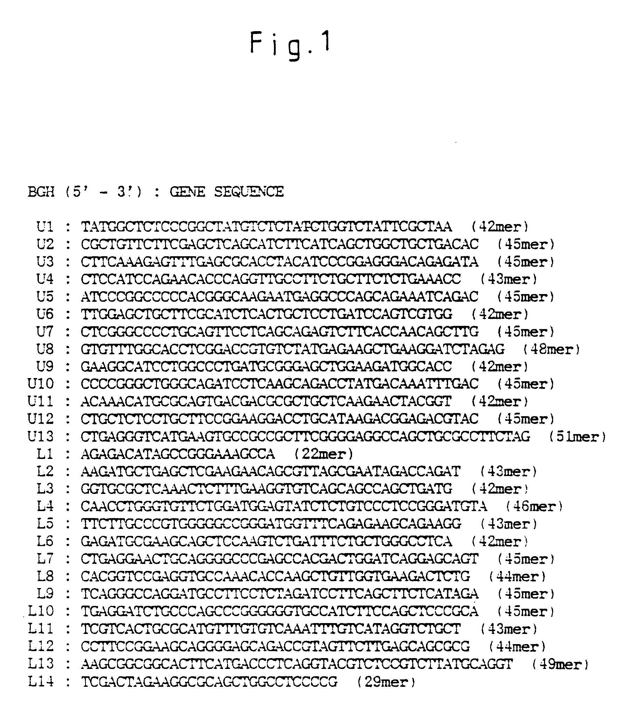

- Fig. 1: shows synthetic oligonucleotides corresponding to a whole bovine growth hormone gene and represented by the base sequence from the 5'-end to the 3'-end.

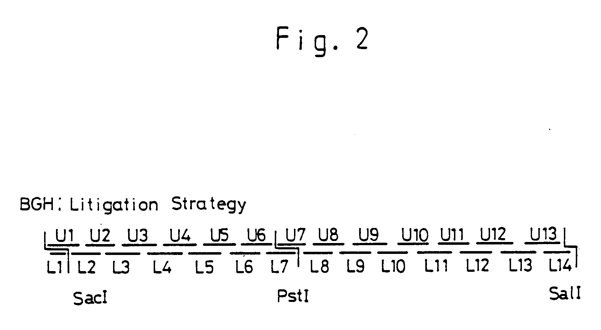

- Fig. 2: is a ligation strategy of oligonucleotides in accordance with Fig 1.
- Fig. 3: is a schematic cloning process of synthetic oligonucleotides to a vector for E.coli.
- Fig. 4: is the base sequence and putative amino acid sequence of the confirmed bovine growth hormone gene.
- Fig. 5: is a cloning process to a vector for E.coli (pBS 100) and a vector for yeast expression (pCl/1) to produce boving growth hormone in yeast.
- Fig. 6: is a reassociation process of a complete bovine growth hormone gene to a vector for expression in E.coli.
- Fig. 7: shows the results confirmed by SDS-polyacrylamide gel electrophoresis of bovine growth hormone produced in yeast cells.
- Fig. 8: shows the results confirmed by SDS-polyacrylamide gel electrophoresis of bovine growth hormone produced in E.coli.

### DETAILED DESCRIPTION OF A PREFERRED EMBODIMENT

The present inventors have altered the 5'-end of the base sequence of bovine growth hormone by selecting amino acid codons preferentially used in yeast cells, based upon the amino acid sequence of bovine growth hormone, reported by Milter et al. [J.Biol. Chem. 255 (1980), 7521) and the total gene was chemically synthesized with a gene synthesizer (Applied Biosystem. Model 380B, USA) according to phosphoramidate's method in order that the NH₂-terminal of the mature bovine growth hormone in the natural state is initiated with ala(Li, C.H. & Ash, L.J. Biol. Chem 203, 419-424).

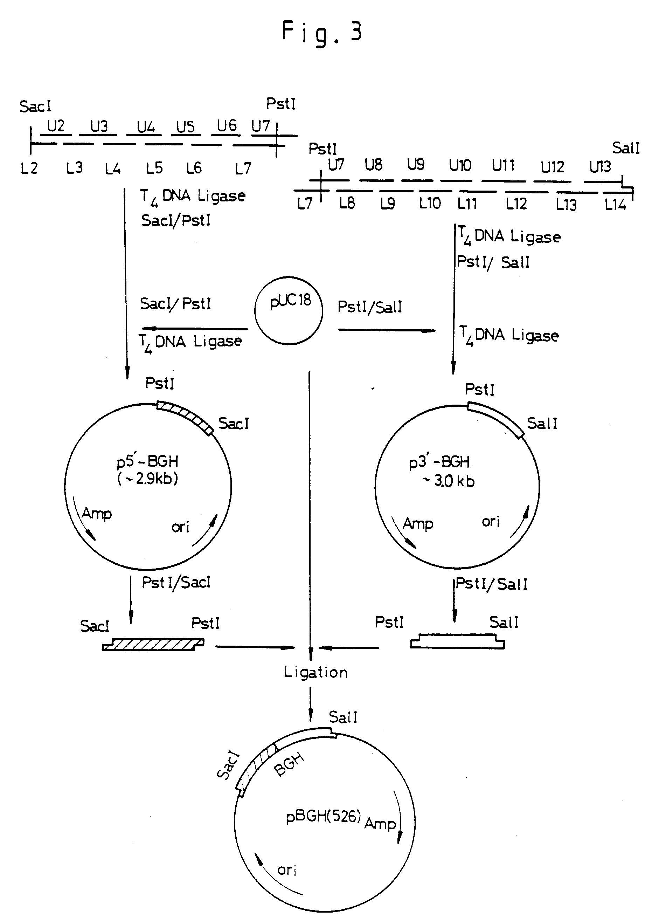

The synthetic oligonucleotides were ligated and cloned to a vecotr for E.coli, pUC18 [Norrander, J. et al. Gene 36 (1983) 101-106] to produce pBGH(526) comprising SalI/SacI restriction fragment of 526 bp (Refer to Fig. 3).

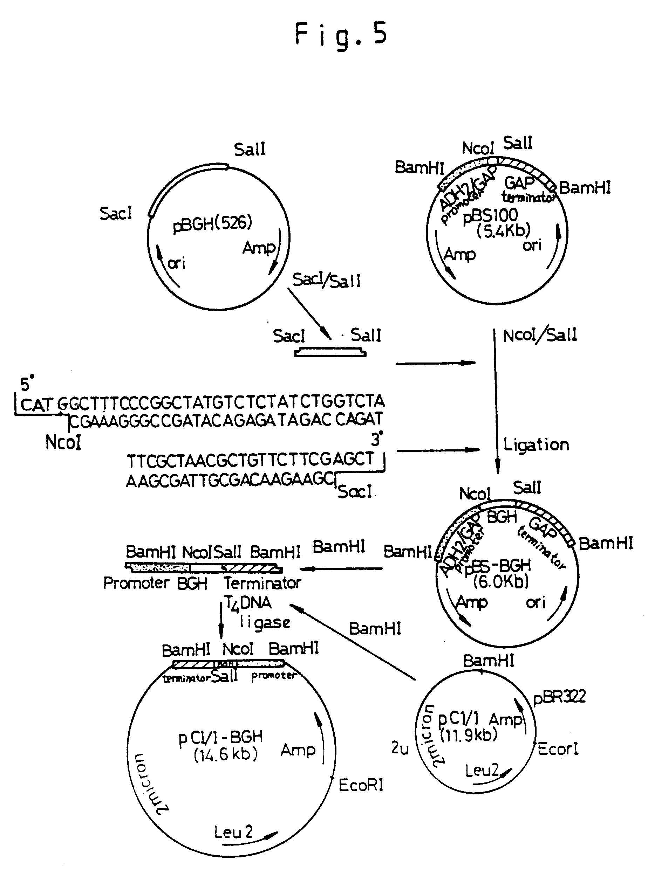

Firstly, a method for the production of bovine growth hormone from yeast cells is as follows ; The clone comprising the bovine growth hormone gene was produced and the SacI/SalI fragment of 526 bp obtained from pBGH(526) with a synthetic adaptor was ligated between the NcoI and SalI sites of a vector having a promoter and a terminator gene, pBS100 [Yeast 2.72^{.} (1986) ; Chiron Corp. Emeryvill, CA 94608, U.S.A] to express in yeast, wherein the adaptor chemically synthesized is comprised of the NcoI restriction site, an initiation codon and an amino acid codon corresponding to the N-terminal region. The resultant vector is pBS-BGH (576) (Refer to Fig. 5).

pBS-BGH(576) was treated with BamHI to separate the BamHI fragment (about 2.7Kb) comprising a promoter, the bovine growth hormone gene and a terminator. The BamHI fragment was inserted into the BamHI restriction site of a yeast expression vector, pC1/1, which is automatically replicated in E.coli and yeast [ATCC 37115 ; Brake et al. Proc. Natl. Acad. Sci USA 81 (1984), 4642] to produce pC1/1-BGH (Refer to Fig. 5).

The above-mentioned DNA is cloned to yeast strain DCO4 [Broach, J.R. & Hicks, J.B. Cell 21, 501(1980) ; Yeast Genetic Stock Center] by means of Hinnen et al's method. The transformed yeast cell was cultured in YEPD medium containing 2% glucose as in Example 4 for 48hrs. Whenever the concentration of glucose is exhausted, the bovine growth hormone is automatically induced and about 500mg bovine growth hormone per liter culture can be produced.

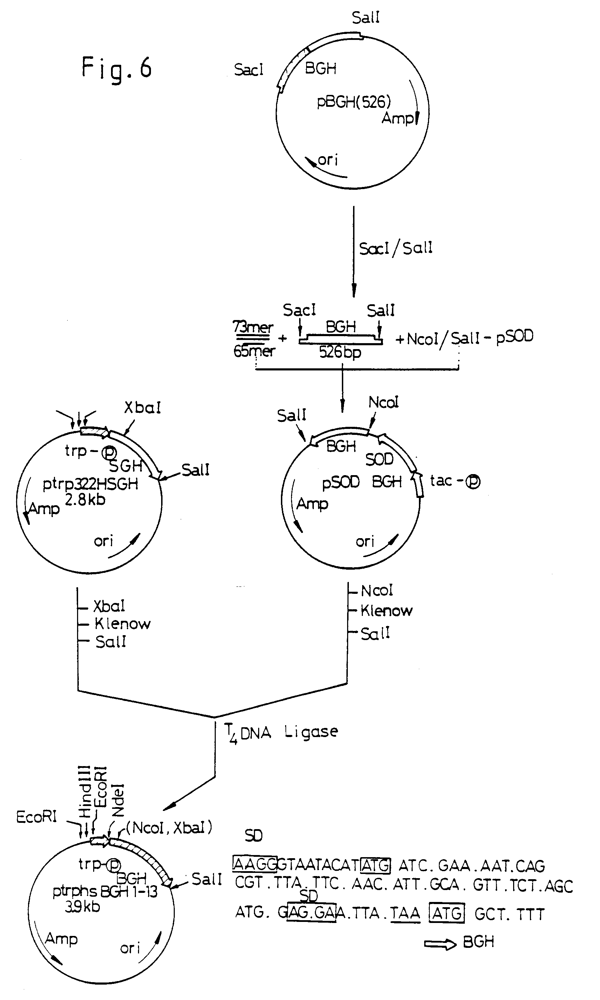

Secondly, a method for production of bovine growth hormone in E.coli is as follows ; Vector pBGH (526) comprising 526 bp and a specifically designed adaptor were cloned to NcoI and SalI restriction sites of vector pSOD to obtain pSOD BGH containing a complete bovine growth hormone gene (Refer to Fig. 6).

In order for bovine growth hormone to be expressed in E.coli, an E.coli expression vector, ptrp322H SGH was cloned and the polycistron method using an expression vector, ptrp322H SGH where salmon growth hormone is mass-expressed, is specifically adopted. The SD(Shine-Dalgarno)sequence, an initiation codon (ATG) in the left side of the salmon growth hormone gene and the region including XbaI restriction site located at the right side from the initiation codon were used. The second SD sequence, the terminating codon (TAA) for stopping synthesis of a part of the salmon growth hormone and the complete bovine growth hormone gene were ligated thereto to maximize the expression of bovine growth hormone.

The characteristic of the above method is as follows ; the inhibition effect which is a result of the formation of a secondary structure of mRNA between the SD sequence and ATG, reported as a problem in case that a foreign protein is expressed in E.coli, was eliminated by using the established gene construction whose expression rate was high.

E.coli W3110 (ATCC 27325, Rockville, MD 20852, U.S.A.) containing an expression vector, ptrphs BGH 1-13 prepared as described above was selected and cultured under the condition of expression. By SDS polyacrylamide gel electrophoresis and densitomer scanning, it was confirmed that more than 30% of the whole E.coli proteins was bovine growth hormone, and also it was proven that it was bovine growth hormone by Western-blotting.

The invention is illustrated by the following Examples, without them limiting its range.

### Example 1 : Ligation and cloning of synthetic oligonucleotides for bovine growth hormone gene

The oligonucleotides (U7-U13/L7-L14) corresponding to the COOH-terminal half of the whole synthetic oligonucleotides were taken the amount of 0.05 OD₂₆₀ respectively, and then separately dried. Four units of T4 polynucleotide kinase were added to the total volume of 30 µl in the presence of buffer solution containing 50mM Tris-HCl (pH 7.5), 1mM ATP, 1mM DTT and 10mM MgCl₂ and each reaction mixture was incubated for 30mins. at 37 °C in order to phosphorylate the 5'-end residue of the oligonucleotides. After all of the oligonucleotides were pooled and treated with an equal volumes of phenol and chloroform mixture, they were precipitated with ethanol. The precipitates were dissolved in 53 µl of buffer solution containing 60mM Tris-HCl (pH 7.5), 1mM DTT and 10mM MgCl₂. The solution was placed in 95 °C water bath and kept at room temperature for 6 hrs. so that its temperature droped slowly. T4 DNA ligase (20 units) and 5 µl of 10mM ATP are added herein and the 5'-and 3'-ends of oligonucleotides were ligated at room temperature for 10 mins. The above solution was treated with phenol and chloroform mixture and precipitated with ethanol.

Ten units each of PstI and SalI restriction enzymes were added to the precipitate in the presence of buffer solution containing 60mM Tris-HCl (pH 7.6) and 10mM NaCl and reacted at 37 °C for 1 hr.

After 7% polyacrylamide gel electrophoresis, a band corresponding to 280-330 bp was cut from the gel. After electroelution, the solution was precipitated with ethanol and the precipitate was dissolved in 20 µl of distilled water.

Three µl of the precipitate and 10ng of a vector, pUC18, cut with PstI and SalI restriction enzymes were ligated in the presence of the ligation buffer containing 60mM Tris-HCl (pH 7.5), 10mM DTT, 10mM MgCl₂, 1mM ATP and 10 units of T4 DNA ligase at 14 °C for 16 hrs.

E.coli JM 103 [BRL, U.S.A., Messing, J. Methods in Enzymology 101, 20-78 (1983)] competent cell was added to the ligation mixture and was transformed according to Hanahan's method [J. Mol. Biol 118, 557 (1983)] at 37 °C overnight.

The recombinant clone containing p3'-BGH was selected from the white clonies according to Birnboim and Doly's method [Nucleic Acid Res. 7, 1513 (1979)].

On the other hand, the oligonuclotides (U2-U7/L2-L7) corresponding to the 5'-end of the whole synthetic oligonucleotides were ligated by the same method as mentioned above and cloned to pUC18 cut with SacI and PstI restriction enzymes to produce p5'-BGH by the method of Fig. 3.

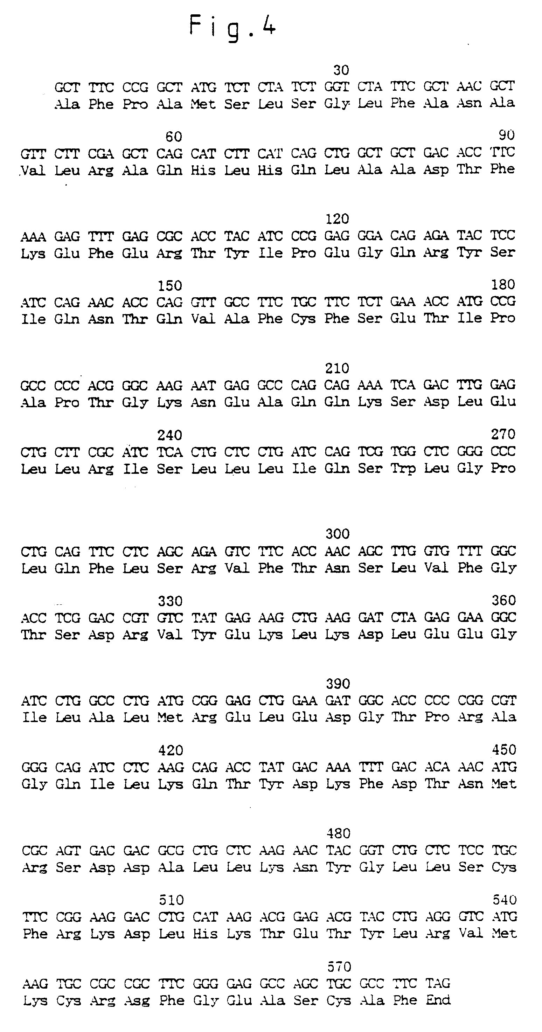

The DNA sequence of p3'-BGH and p5'-BGG were confirmed by the method of Sanger's dideoxy sequencing [Proc. Natl. Acad. Sci. USA 74, 5473-5477 (1977)] (Refer to Fig. 4).

The PstI-SalI restriction fragment from p3' -BGH and the SacI-PstI restriction fragment from p5'-BGH were respectively separated from 1% agarose gel and ligated to a vector, pUC18, cut with SacI and SalI restriction enzymes to produce pBGH (526) (Refer to Fig. 3).

### Example 2 : Manipulation of synthetic bovine growth hormone gene for exprssion in yeast cells

The deficient part corresponding to the 19 amino acids of the 5'-end of the complete bovine growth hormone gene in pBGH (526) was synthesized by DNA synthesizer in order for the mature gene to be cloned and expressed in yeast cells. The NcoI restriction site, an initiation codon and nucleotides corresponding to 18 amino acids were synthesized by selecting codons preferentially used in yeast cells (Refer to Fig. 5).

The process for cloning is as follows:pBGH (526) was treated with SacI and SalI restriction enzymes to obtain a 526 bp restriction fragment. The separated fragment and the above-mentioned synthetic adaptor were inserted into NcoI and SalI restriction sites of pBS100 containing an inducible promoter and a terminator [Chiron Corp. Emeryville, CA 94608, U.S.A. ; Yeast 2, 72 (1986)].

One µl of each phosphorylated adaptor, 3µl (30ng) of SacI/SalI fragment of separated bovine growth hormone and 1µl (7ng) of vector pBS100, cut with NcoI and SalI restriction enzymes were mixed, kept at 65 °C for 15 mins. and cooled slowly at room temperature. Two µl of 10mM ATP, 2µl of 10-fold concentrated ligation reaction buffer, 20 units of ligase and 8µl of distilled water were added and reacted at 14 °C for 16 hrs. As described in Example 1, the mixture was transformed to E.coli HB 101 (ATCC 33694) and pBS-BGH (576) comprising a promoter, the complete bovine growth hormone gene and a terminatior was produced (Refer to Fig. 5).

The vector, pBS100, contains the hybrid promoter of promoter of alcohol dehydrogenase II and glyceraldehyde 3' -phosphate dehydrogenase and which can be induced whenever glucose is depleted.

The pBS-BGH (576) was treated with BamHI restriction enzyme to separate the BamHI restriction fragment of about 2,700 base pairs, containing a hybrid promoter, the bovine growth hormone and a terminator of glyceraldehyde 3'-phosphate dehydrogenase. The BamHI restriction fragment was inserted into the BamHI restriction site of the expression vector pC1/1 (ATCC 37115), which is able to be replicated in yeast cell, to obtain pC1/1-BGH (Refer to Fig. 5).

Ten µg of pC1/1-BGH DNA was protoplast-transformed to yeast strain DCO4 [Yeast Genetic Stock Center, University of California, Berkeley, CA, USA ; Broach, J.R. & Hicks, J. B. Cell 21 501 (1980)] according to Hinnen's method [Proc. Natl, Acad. Sci. USA 75 1929, (1978)] and plated on agar-plates without leucine, comprising 6.7g of Yeast Nitrogen Base without amino acids, 182g of sorbitol 2% glucose, 0.25g of Leu-deficient amino acid supplements and 20g of Bactoagar per liter medium.

After culture at 30 °C for 5 days, transformants were obtained.

### Example 3 : Manipulation of bovine growth hormone gene to an E. coli expression vector

Codons preferentially used in E.coli were selected between the SacI restriction site of bovine growth hormone gene and an initiation codon ATG. A termination codon, TAA and independent SD sequence for a polycistronic construction with pSOD designed so as that the secondary structure of mRNA was not formed in the vicinity of the SD sequence and initiation codon ATG. The adaptor were an upper strand of 73 mer (5'-CATG GAG GAA 
TTA TAA ATG GCT TTT CCG GCT ATG TCT CTA TCT GGT CTA TTC GCT

(3'
and a lower strand of 65 mer 
(3'
CTC CTT AAT ATT TAC CGA AAA GGC CGA TAC AGA GAT AGA CCA GAT
AAG CGA TTG CGA CAA GAA GC-5')
synthesized by the DNA synthesizer. The synthetic adaptor and the SacI-SalI restriction fragment of 526 bp obtained from pBGH(526) were inserted to a vector pSOD, cut with NcoI and SalI restriction enzymes to obtain the pSOD BGH containing the complete bovine growth hormone gene(Refer to Fig. 6).

In order that the bovine growth hormone is mass-expressed in E.coli using the trp promoter, a polycistron method in which the complete bovine growth hormone gene is arranged behind the NH₂-terminal region of the salmon growth hormone gene mass-expressed in E.coli, were developed and executed as in Fig. 6.

That is, 5 µg of an expression vector, ptrp322H SGH DNA in which the salmon growth hormone is mass-expressed was reacted with 10 units of XbaI restriction enzyme in a buffer solution containing 50mM Tris -HCl (pH 8.0), 10mM MgCl₂ and 50mM NaCl at 37 °C for 1 hr. The solution was treated with phenol and chloroform mixture and precipitated with ethanol. The precipitate was dried and dissolved in 15.5 µl of distilled water.

Then dNTP (dGTP, dCTP, dATP, dTTP) was added so as that the final concentration in 20 µl of the total reactant became 250 µM and the solution was reacted with 2.5 units of Klenow enzyme in a buffer solution containing 50mM Tris-HCl (pH 8.0), 10mM MgCl₂ and 50mM NaCl at room temperature for 20mins. in order that the site, cut with XbaI restriction enzyme of salmon growth hormone gene was made blunt-end. After the activity of Klenow enzyme was inactivated by keeping the above solution at 95 °C for 2 mins, 10 units of SalI restriction enzyme, 6.5 µl of 1M NaCl and 20.5 µl of distilled water were added and incubated at 37 °C for 1 hr. A DNA fragment of 2.4 Kb was separated from 1% agarose gel.
The electroeluted DNA was dissolved in 15 µl of TE comprising 10mM Tris-HCl (pH 7.5) and 1mM EDTA.

On the other hand, in order to obtain the DNA fragment of the complete bovine growth hormone gene and independent SD sequence, 10 µg of pSOD BGH DNA prepared by the above-mentioned method was rected with 12 units of NcoI restriction enzyme in a buffer solution comprising 150mM NaCl, 6mM Tris-HCl (pH 7.9) and 6mM MgCl₂ at 37 °C for 1 hr. The reactant was treated with phenol and chloroform mixture and precipitated with ethanol. The precipitate was dried and dissolved in 31.5 µl of distilled water. dNTP (dGTP, dCTP, dATP, dTTP) was added so as that the final concentration became 250 µM, and the solution was reacted with 2.5 units of Klenow enzyme in a bufer solution containing 50mM Tris-HCl (pH 8.0), 10mM MgCl₂ and 50mM NaCl at room temperature for 20 mins. After the solution was kept at 95 °C for 2mins, 20 units of SalI restriction enzyme, 7 µl of 1M NaCl and 10 µl of distilled water were added and then incubated at 37 °C for 1 hr. The DNA fragment of 595 dp was isolated from 1% agarose gel electrophoresis, electroelution, and dissolved in 15 µl of TE.

The 10ng of vector DNA of 2.4 Kb obtained by treating ptrp 322 H SGH DNA with XbaI, Klenow enzyme and SalI restriction enzyme and 50ng of DNA containing the complete bovine growth hormone gene were ligated with 20 units of T4 DNA ligase in a buffer solution containing 60mM Tris-HCl (pH 7.5), 1mM DTT and 10mM MgCl₂ at 14 °C for 16hrs. Then it was cloned to E.coli HB101 according to Hanahan's method to obtain ptrphsBGH 1-13. The obtained vector, ptrphsBGH 1-13 was cloned to E.coli W 3110 (ATCC 27325).

### Example 4 : Cultivation of yeast for producing bovine growth hormone and its identification

Each 3ml of yeast cells transformed with vector pC1/1-BGH was cultured in a medium without leucine (6.7g Yeast Nitrogen Base without amino acids, 0.25g of Leu-deficient amino acids supplements and 2% glucose per liter medium) at 30 °C for 24 hrs. YEPD culture medium (100 ml) comprising 2% peptone, 1% yeast extract and 2% glucose was inoculated with 1ml of overnight culture, and further cultured at 30 °C for 24 hrs. 40ml of ethanol was added thereto and the solution was further cultured for 24hrs.

The resultant OD₆₅₀ was about 40. That OD₆₅₀ of 10 was taken, centrifuged, and dissolved in 400 µ1 of a buffer solution containing 10mM Tris-HCl (pH 7.5), 1mM EDTA, 2mM phenyl methyl sulfonyl fluoride (PMSF) and 8M urea and glass beads having a diameter of 0.45 mm were added and vortexed vigorously. After rupturing the cell wall and allowing the bovine growth hormone to elute into the buffer solution, 4 µ1 of eluted solution was executed by electrophoresis on 12.5% SDS polyacrylamide gel.

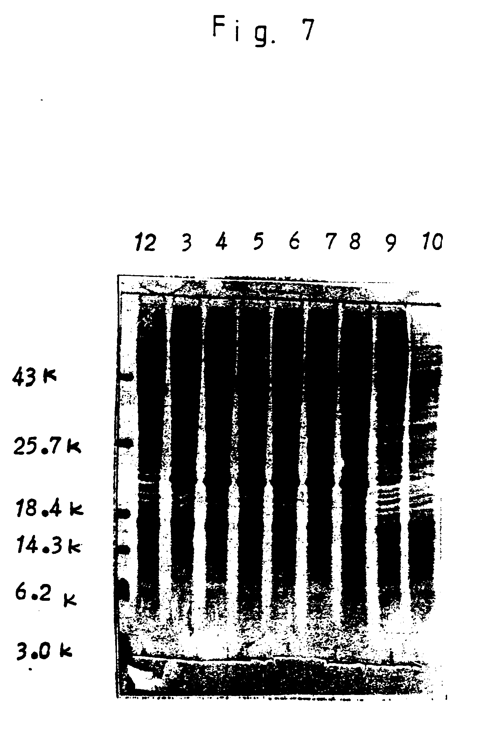

The results are represented in Fig. 7;
- Lane 1: represents molecular weight of marker proteins from BRL company.
- Lane 2: represents total proteins of yeast cell transformed with vector pC1/1-BGH before induction with ethanol.
- Lanes 3-8: represent total proteins of yeast cell transformed with vetor pC1/1-BGH after induction with ethanol.
- Lane 10: represents total proteins of yeast cells transformed with vector pC1/1 without bovine growth hormone gene.

As seen in lane 3 of Fig. 7, the bovine growth hormone expressed at a band about 22Kd in an amount corresponding to 20% of the total proteins by a densitometric scanning of the protein gel.

Example 5 : Mass-expression of boving growth hormone in
E.coli W 3110 and confirmation by SDS poly-
acrylamide gel electrophoresis and Western-
blotting

E.coli W 3110 comprising the expression vector, ptrphs BGH 1-13 was cultured on LB medium with 40µg/ml of ampicillin at 37 °C for 16 hrs with shaking, and at the point that OD₆₅₀ of 0.5, 60µg/ml of IAA (Indole acrylic acid) was added. The solution was further cultured at 37 °C for 18hrs. The cultured cells corresponding to OD₆₅₀ of 1.0 was centrifuged and dissolved in 100 µ1 of Laemmli sample buffer solution [Laemmli U.K. Nature 227, 680 (1970)]. The solution was heated at 100 °C for 5 mins. and then 10 µ1 of it was run to 12.5% SDS polyacrylamide gel electrophoresis.

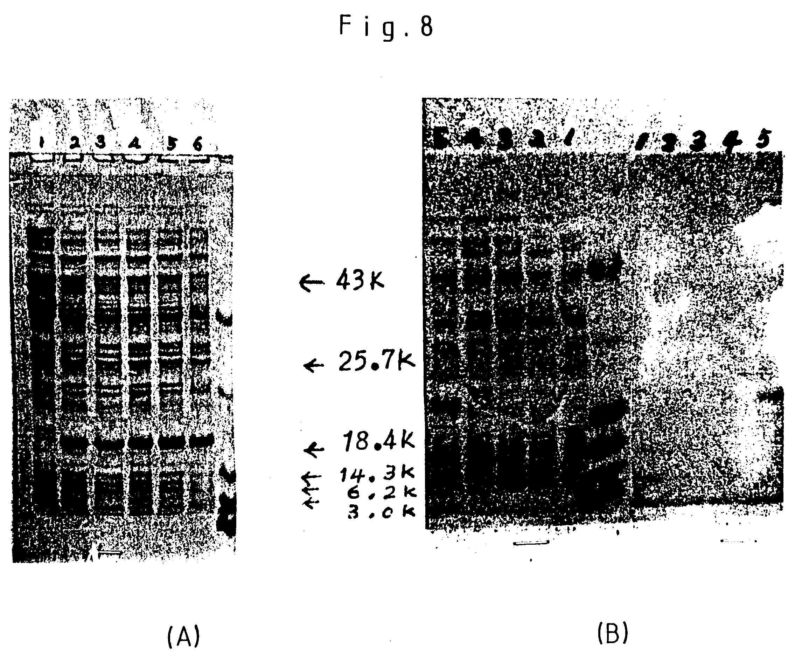

The results are represented in (A) of Fig 8 ;
(A) represents the results confirmed by SDS-polyacrylamide gel after the expression strain of bovine growth homone was cultured under the conditions described above.
   - Lane 1: represents the uninduced E.coli W3110 with ptrphs BGH.
   - Lanes 2-6: represent the induction by IAA.

As shown in (A) of Fig. 8, a protein band of 22Kd which can not be seen in uninduced cells appears to be an amount of more than 30% of total proteins of E.coli.

Western-blotting was done by using monoclonal antibody against bovine growth hormone to which horse radish peroxidase conjugated [Accurate Chemical Scientific Corp. U.S.A.] according to Burnette et al.'s method [Anal. Biochem. 112, 195 (1981)] and the results are represented in (B) of Fig 8.
(B) represents the result confirmed by Western-blotting of bovine growth hormone expressed in E.coli.
   - Lanes 1-3: represent the strain which does not contain bovine growth hormone gene.
   - Lane 4: represents the uninduced cells.
   - Lane 5: represents the induction by IAA.

As seen in (B) of Fig. 8, the immunoactivity appears at the same location as the protein band which appears in molecular weight, 22 kilodaltons.

That is, the present inventors confirmed that the bovine growth hormone expressed has immunoactivity.

M9 medium comprises 40mM K₂HPO₄, 22mM KH₂PO₄, 8.5mM NaCl, 18.7mM NH₄Cl, 1mM MgSO₄, 0.1mM CaCl₂, 10µg/ml Vit B₁, 0.4% CAA(Casamino acid), 1% glucose and 40µg/ml ampicillin.

## Claims

1. A method for the production of bovine growth hormone, comprising (a) synthesizing oligonucleotides corresponding to the amino acid sequence of bovine growth hormone, (b) ligating the oligonucleotides and cloning the ligated oligonucleotides into an E. coli vector to produce a bovine growth hormone coding sequence lacking a portion of the N-terminus, (c) inserting the bovine growth hormone coding sequence and an N-terminal synthetic adaptor having the nucleotide sequence:- 
5'
CATGGCTTTCCCGGCTATGTCTCTATCTGGTCTA
CGAAAGGGCCGATACAGAGATAGAC CAGAT
NcoI site 3'
TTCGCTAACGCTGTTCTTCGAGCT
AAGCGATTGCGACAAGAAGC
SacI. site
 into a vector with a promoter and a terminator to form a cassette containing a promoter-bovine growth hormone construct followed by a stop codon, (d) inserting the cassette into a yeast expression vector and (e) expressing the bovine growth hormone gene in yeast cells.

2. A method for the production of bovine growth hormone as claimed in Claim 1, wherein the synthetic oligonucleotides have SacI, PstI and SalI restriction sites.

3. A method for the production of bovine growth hormone as claimed in Claim 1 and Claim 2 wherein the bovine growth hormone coding sequence lacking a portion of the N-terminus is produced by cloning the C terminal fragment with the PstI/SalI sites and the N-terminal fragment with the SacI/PstI sites into a vector, preferably pUC18, comprising PstI, SacI and SalI restriction sites, respectively.

4. A method for the production of bovine growth hormone as claimed in any one of the preceding Claims wherein the resulting vector for expression in yeast is a vector pC1/1-BGH obtained by treating the yeast expression vector pC1/1 with BamHI and then inserting the cassette of promoter-bovine growth hormone construct comprising an initiation amino-acid codon-terminator to it.

5. A method for the production of bovine growth hormone comprising (a) synthesizing oligonucleotides having SacI, PstI and SalI restriction sites corresponding to the amino acid sequence of bovine growth hormone, (b) ligating the oligonucleotides and cloning the ligated oligonucleotides into an E. coli vector to produce a bovine growth hormone coding sequence (c) cloning the partial bovine growth hormone coding sequence and an N-terminal synthetic adaptor having the upperstrand nucleotide sequence:- 
73 mer (5'-CATG GAG GAA TTA
TAA ATG GCT TTT CCG GCT ATG TCT CTA TCT GGT CTA TTC GCT
AAC GCT GTT CTT CGA GCT-3')
 and a lower strand of 65mer 
(3'-CTC CTT AAT ATT TAC CGA AAA GGC CGA TAC AGA GAT AGA
CCA GAT AAG CGA TTG CGA CAA GAA GC-5')
 into an E. coli vector to obtain the bovine growth hormone coding sequence ligated with the synthetic adaptor (d) inserting the obtained bovine growth hormone coding sequence into an E. coli expression vector capable of expressing salmon growth hormone under the control of a trp promoter and (e) expressing the bovine growth hormone gene in E. coli.

6. A method for the production of bovine growth hormone as claimed in Claim 5 wherein the synthetic adaptor has a termination codon TAA and an independent Shine Dalgarno sequence before an initiation codon ATG of the bovine growth hormone coding sequence and prevents formation of the secondary structure of mRNA between the SD sequence and the initiation codon.

7. A method for the production of bovine growth hormone as claimed in Claims 5 and 6 wherein the bovine growth hormone coding sequence ligated the synthetic adaptor is obtainable by cloning the SacI-SalI fragment comprising 526bp of the bovine growth hormone coding sequence and a synthetic adaptor a vector pSOD, treated with NcoI and SalI restriction enzymes to obtain the vector pSOD-BGH.

8. A method for the production of bovine growth hormone as claimed in Claims 5, 6 and 7 wherein the resulting E. coli expression vector is vector ptrphs BGH 1-13 produced by (a) treating vector pSOD-BGH with NcoI and SalI restriction enzymes to separate the bovine growth hormone coding sequence and (b) cloning the separated fragment into the E. coli expression vector ptrp322H SGH in which a part of the salmon growth hormone coding sequence was eliminated by treatment with XbaI and SalI restriction enzymes.

9. A method of production of bovine growth hormone as claimed in any one of Claims 5 to 8 wherein the expression in E. coli is effected using a Shine-Dalgarno sequence and an initiation codon ATG in front of the salmon growth hormone gene.

10. A method of production of bovine growth hormone as claimed in any one of Claims 5 to 9 wherein the expression in E. coli is effected by ligating the bovine growth hormone coding sequence with a synthetic adaptor comprising a Shine-Dalgarno sequence and a terminating codon TAA to the NH₂-terminal region of salmon growth hormone gene in order to make a polycistronic construct.

## Patentansprüche

1. Verfahren zur Herstellung von Rinderwachstumshormon, welches (a) die Synthese von Oligonucleotiden, die der Aminosäuresequenz von Rinderwachstumshormon entsprechen, (b) Ligation der Oligonucleotide und Einklonierung der ligierten Oligonucleotide in einen E. coli-Vektor, um eine Rinderwachstumshormon-Codierungssequenz zu erzeugen, der ein Teil des N-Terminus fehlt, (c) Insertion der RinderwachstumshormonCodierungssequenz und eines N-terminalen synthetischen Adaptors mit der Nucleotidsequenz: 
5'
CATGGCTTTCCCGGCTATGTCTCTATCTGGTCTA
CGAAAGGGCCGATACAGAGATAGACCAGAT
NcoI-
STELLE
3'
TTCGCTAACGCTGTTCTTCGAGCT
AAGCGATTGCGACAAGAAGC
SacI-
STELLE
 in einen Vektor mit einem Promotor und einem Terminator, um eine ein Promotor-Rinderwachstumshormon-Konstrukt, gefolgt von einem Stopcodon, enthaltende Kassette zu bilden, (d) Insertion der Kassette in einen Hefe-Expressionsvektor und (e) Expression des Rinderwachstumshormon-Gens in Hefezellen umfaßt.

2. Verfahren zur Herstellung von Rinderwachstumshormon nach Anspruch 1, bei welchem die synthetischen Oligonucleotide SacI-, PstI- und SalI-Restriktionsstellen aufweisen.

3. Verfahren zur Herstellung von Rinderwachstumshormon nach Anspruch 1 und 2, bei welchem die RinderwachstumshormonCodierungssequenz, der ein Teil des N-Terminus fehlt, durch Einklonierung des C-terminalen Fragments mit den PstI/SalI-Stellen und des N-terminalen Fragments mit den SalI/PstI-Stellen in einen Vektor, vorzugsweise pUC18, der PstI-, SacI- bzw. SalI-Restriktionsstellen umfaßt, erzeugt wird.

4. Verfahren zur Herstellung von Rinderwachstumshormon nach einem der vorhergehenden Ansprüche, bei welchem der erhaltene Vektor für eine Expression in Hefe ein Vektor pC1/1-BGH ist, der durch Behandlung des Hefe-Expressionsvektors pC1/1 mit BamHI und anschließende Insertion der Kassette des Promotor-Rinderwachstumshormon-Konstrukts mit einem Initiations-Aminosäurecodon-Terminator in diesen erhalten wird.

5. Verfahren zur Herstellung von Rinderwachstumshormon, welches (a) die Synthese von Oligonucleotiden mit SacI-, PstI- und SalI-Restriktionsstellen, die der Aminosäuresequenz von Rinderwachstumshormon entsprechen, (b) Ligation der Oligonucleotide und Einklonierung der ligierten Oligonucleotide in einen E. coli-Vektor, um eine Rinderwachstumshormon-Codierungssequenz zu erzeugen, (c) Einklonierung der teilweisen Rinderwachstumshormon-Codierungssequenz und eines N-terminalen synthetischen Adaptors mit der stromaufwärtigen Nucleotidsequenz: 
73-mer (5'-CATG GAG GAA TTA TAA ATG GCT TTT CCG GCT ATG TCT
CTA TCT GGT CTA TTC GCT AAC GCT GTT CGA GCT-3')
 und einem stromabwärtigen Strang des 
65-mer (3'-CTC CTT AAT ATT TAC CGA AAA GGC CGA TAC AGA GAT
AGA CCA GAT AAG CGA TTG CGA CAA GAA GC-5')
 in einen E. coli-Vektor, um die mit dem synthetischen Adaptor ligierte Rinderwachstumshormon-Codierungssequenz zu erhalten, (d) Insertion der erhaltenen Rinderwachstumshormon-Codierungssequenz in einen E. coli-Expressionsvektor, der Lachswachstumshormon unter der Kontrolle eines trp-Promotors exprimieren kann, und (e) Expression des Rinderwachstumshormon-Gens in E. coli.

6. Verfahren zur Herstellung von Rinderwachstumshormon nach Anspruch 5, bei welchem der synthetische Adaptor ein Terminationscodon TAA und eine unabhängige Shine-Dalgarno-Sequenz vor einem Initiationscodon ATG der Rinderwachstumshormon-Codierungssequenz aufweist und die Bildung der Sekundärstruktur von mRNA zwischen der SD-Sequenz und dem Initiationscodon verhindert.

7. Verfahren zur Herstellung von Rinderwachstumshormon nach Anspruch 5 und 6, bei welchem die an den synthetischen Adaptor ligierte Rinderwachstumshormon-Codierungssequenz durch Einklonierung des SacI-SalI-Fragments, das 526 bp der Rinderwachstumshormon-Codierungssequenz und einen synthetischen Adaptor umfaßt, in einen Vektor pSOD, der mit NcoI- und SalI-Restriktionsenzymen behandelt worden ist, um den Vektor pSOD-BGH zu ergeben, erhalten werden kann.

8. Verfahren zur Herstellung von Rinderwachstumshormon nach Anspruch 5, 6 und 7, bei welchem der erhaltene E. coli-Expressionsvektor der Vektor ptrphs BGH 1-13 ist, der durch (a) Behandlung des Vektors pSOD-BGH mit NocI- und SalI-Restriktionsenzymen, um die Rinderwachstumshormon-Codierungssequenz abzutrennen, und (b) Einklonierung des abgetrennten Fragments in den E. coli-Expressionsvektor ptrp322H SGH, in dem ein Teil der Lachswachstumshormon-Codierungssequenz durch Behandlung mit XbaI- und SalI-Restriktionsenzymen eliminiert wurde, erzeugt wird.

9. Verfahren zur Herstellung von Rinderwachstumshormon nach einem der Ansprüche 5 bis 8, bei welchem die Expression in E. coli unter Verwendung einer Shine-Dalgarno-Sequenz und eines Initiationscodons ATG vor dem Lachswachstumshormon-Gen bewirkt wird.

10. Verfahren zur Herstellung von Rinderwachstumshormon nach einem der Ansprüche 5 bis 9, bei welchem die Expression in E. coli durch Ligation der Rinderwachstumshormon-Codierungssequenz mit einem synthetischen Adaptor, der eine Shine-Dalgarno-Sequenz und ein Terminationscodon TAA umfaßt, mit der NH₂-terminalen Region des Lachswachstumshormon-Gens bewirkt wird, um ein polycistronisches Konstrukt herzustellen.

## Revendications

1. Un procédé de production de l'hormone de croissance bovine comprenant:
(a) la synthèse d'oligonucléotides correspondant à la séquence d'acides aminés de l'hormone de croissance bovine;
(b) la ligation des oligonucléotides et le clonage des oligonucléotides liés dans un vecteur de E. coli de façon à produire une séquence codant pour l'hormone de croissance bovine ne comportant par la partie N-terminale;
(c) l'insertion de la séquence codant pour l'hormone de croissance bovine et d'un adaptateur synthétique N-terminal ayant la séquence nucléotide suivante: 
5'-CATGGCTTTCCCGGCTATGTCTCTATCTGGTCTATTCGCTAACGCTGTTCT
CGAAAGGGCCGATACAGAGATAGACCAAACAAGCGATTGCGACAAGA
site NcoI
TCGAGCT - 3'
AGC-
site SacI
 dans un vecteur comprenant un promoteur et un terminateur pour former une cassette contenant une construction promoteur-hormone de croissance bovine suivie d'un codon stop;
(d) l'insertion de la cassette dans un vecteur d'expression de levure; et
(e) l'expression du gène de l'hormone de croissance bovine dans les cellules de levure.

2. Un procédé de production de l'hormone de croissance bovine selon la revendication 1, caractérisé en ce que les oligonucléotides synthétiques comportent des sites de restriction SacI, PstI et SalI.

3. Un procédé de production de l'hormone de croissance bovine selon les revendications 1 et 2, caractérisé en ce que la séquence codant pour l'hormone de croissance bovine et ne comportant pas la partie N-terminale est produite par clonage du fragment C-terminal avec les sites PstI/SalI et du fragment N-terminal avec les sites SacI/PstI dans un vecteur qui correspond de préférence à pUC18 et qui comprend respectivement les sites de restriction PstI, SacI et SalI.

4. Un procédé de production de l'hormone de croissance bovine selon l'une quelconque des revendications précédentes, caractérisé en ce que le vecteur résultant permettant l'expression chez la levure est un vecteur pC1/1-BGH, obtenu par traitement d'un vecteur d'expression de levure pC1/1 avec BamHI, puis insertion dans ce dernier de la cassette permettant la construction promoteur-hormone de croissance bovine comprenant un codon d'initiation et un terminateur.

5. Un procédé de production de l'hormone de croissance bovine comprenant:
(a) la synthèse d'oligonucléotides comportant les sites de restriction SacI, PstI et SalI correspondants à la séquence d'acides aminés de l'hormone de croissance bovine;
(b) la ligation des oligonucléotiques et le clonage des oligonucléotides liés dans un vecteur d'E. coli pour produire une séquence codant pour l'hormone de croissance bovine;
(c) le clonage de la séquence codant pour une hormone de croissance bovine partielle et d'un adaptateur synthétique N-terminal comportant un brin supérieur ayant la séquence nucléotide suivante: 
73 mer (5'-CATG GAG GAA TTA TAA ATG GCT TTT CCG GCT
ATG TCT CTA TCT GGT CTA TTC GCT AAC GCT GTT CTT CGA
(3'-CTC CTT AAT
 et un brin inférieur de 65 mer 
(3'-CTC CTT AAT
ATT TAC CGA AAA GGC CGA TAC AGA GAT AGA CCA GAT AAG CGA
TTG CGA CAA GAA GC-5')
 dans un vecteur d'E. coli de façon à obtenir une séquence codant pour l'hormone de croissance bovine liée avec l'adaptateur synthétique;
(d) l'insertion de la séquence codant pour l'hormone de croissance bovine obtenue dans un vecteur d'expression d'E. coli capable d'exprimer l'hormone de croissance du saumon sous le contrôle d'un promoteur trp; et
(e) l'expression du gène de l'hormone de croissance bovine dans E. coli.

6. Un procédé de production de l'hormone de croissance bovine selon la revendication 5, caractérisé en ce que l'adaptateur synthétique comporte un codon de terminaison TAA et une séquence indépendante Shine-Dalgarno située avant le codon d'initiation ATG de la séquence codante pour l'hormone de croissance bovine et empêche la formation de la structure secondaire de l'ARN messager entre la séquence SD et le codon d'initiation.

7. Un procédé de production de l'hormone de croissance bovine selon les revendications 5 et 6, caractérisé en ce que la séquence codant pour l'hormone de croissance bovine liée à l'adaptateur synthétique peut être obtenue par clonage du fragment SacI-SalI comprenant 526 paires de base de la séquence codant pour l'hormone de croissance bovine et d'un adaptateur synthétique dans le vecteur pSOD qui a été traité par les enzymes de restriction NcoI et SalI de façon à obtenir le vecteur pSOD-BGH.

8. Un procédé de production de l'hormone de croissance bovine selon les revendications 5, 6 et 7, caractérisé en ce que le vecteur d'expression d'E. coli résultant est un vecteur ptrphs BGH 1-13 produit par:
(a) traitement du vecteur pSOD-BGH avec les enzymes de restriction NcoI et SalI pour séparer la séquence codant pour l'hormone de croissance bovine; et
(b) le clonage du fragment séparé dans un vecteur d'expression d'E. coli ptrp322H SGH dans lequel une partie de la séquence codante pour l'hormone de croissance de saumon est éliminée par traitement avec des enzymes de restriction XbaI et SalI.

9. Un procédé de production de l'hormone de croissance bovine selon l'une quelconque des revendications 5 à 8, caractérisé en ce que l'expression dans E. coli est effectuée en utilisant la séquence Shine-Dalgarno et un codon d'initiation ATG qui sont situés en amont du gène de l'hormone de croissance de saumon.

10. Un procédé de production de l'hormone de croissance bovine selon l'une quelconque des revendications 5 à 9, caractérisé en ce que l'expression dans E. coli est obtenue par ligation de la séquence codant pour l'hormone de croissance bovine avec un adaptateur synthétique comprenant la séquence Shine-Dalgarno et un codon de terminaison TAA dans la région NH₂-terminale du gène de l'hormone de croissance du saumon de façon à obtenir une construction polycistronique.
